(19)

Europäisches
Patentamt
European
Patent Office
Office européen
des brevets

(11) **EP 2 708 570 A1**

(12) **EUROPEAN PATENT APPLICATION**

(43) Date of publication:
**19.03.2014 Bulletin 2014/12**

(51) Int Cl.:
**C08H 1/06** *(2006.01)*   **C08J 3/24** *(2006.01)*
**A61K 47/42** *(2006.01)*   **A61K 9/16** *(2006.01)*

(21) Application number: **13184709.7**

(22) Date of filing: **17.09.2013**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: **18.09.2012  JP 2012203806
18.09.2012  JP 2012203807**

(71) Applicant: **NITTO DENKO CORPORATION**
**Ibaraki-shi, Osaka 567-8680 (JP)**

(72) Inventors:
• **MIURA, Chieko**
  **Ibaraki-shi, Osaka 567-8680 (JP)**

• **MAKABE, Miki**
  **Ibaraki-shi, Osaka 567-8680 (JP)**
• **ABE, Eriko**
  **Ibaraki-shi, Osaka 567-8680 (JP)**
• **SUGAWARA, Shinji**
  **Ibaraki-shi, Osaka 567-8680 (JP)**
• **YOSHIKAWA, Toshiyuki**
  **Ibaraki-shi, Osaka 567-8680 (JP)**

(74) Representative: **Grünecker, Kinkeldey,**
  **Stockmair & Schwanhäusser**
  **Leopoldstrasse 4**
  **80802 München (DE)**

(54) **Gelatin Particle and Use Thereof, and Device for Administration of Physiologically Active Substance**

(57)     The present invention relates to a gelatin particle, which is a thermally crosslinked non-porous spherical gelatin particle having a circularity of 0.8 or more and a dry particle diameter of 20 to 1,600 $\mu$m. The average volume swelling ratio of the gelatin particle in a case where the gelatin particle is immersed in physiological saline at 23°C is from 200 to 2,000% relative to an average volume of the gelatin particle in a dried state. As for a swollen gelatin particle, it is preferable that the gelatin used has a jelly strength of 80 to 120 g and the swollen gelatin particle has a particle diameter of 50 to 2,000 $\mu$m. The gelatin particle can be used for blood vessel embolization and also for controlled release of physiologically active substance.

FIG. 1

A MICROSCOPIC IMAGE OF THE GELATIN PARTICLE
OF EXAMPLE 1 IN A DRY STATE

1000μm

EP 2 708 570 A1

**Description**

FIELD OF THE INVENTION

The present invention relates to a gelatin particle and a gelatin particle for controlled release of a physiologically active substance, and a device for administration of a physiologically active substance. Specifically, it relates to a non-porous spherical gelatin particle having a specific volume swelling ratio and a gelatin particle wherein a physiologically active substance is dissolved and retained in this gelatin particle, and a device wherein a gelatin particle containing a physiologically active substance dissolved, impregnated, and retained therein is dispersed together with physiological saline in a syringe.

BACKGROUND OF THE INVENTION

Gelatin is a protein obtained by a thermal treatment of collagen and subsequent extraction and is a familiar material which is utilized in various fields, for example, is used as a gelling agent in foods such as jelly and pharmaceuticals and is used in industrial uses such as adhesives and films. Of these various uses, since highly purified gelatin and collagen used in medical uses are excellent in biocompatibility and are decomposed in a living body and absorbed into the body, the gelatin and collagen have been widely utilized in products such as an embolic material for liver cancer, a sponge material for hemostasis, and a capsule material for oral administration.

For example, Patent Document 1 discloses an invention wherein gelatin as a biocompatible substance is made water-insoluble and is transformed into a porous particle and there is a description that the particle is useful for embolization treatment and as a support for medical preparations. Since the porous particle is impregnated in its porous part using physiological saline or a solution of a pharmaceutical or the like as an impregnating solution, variation of the particle diameter is small before and after the impregnation and, for example, in the case of its use as an embolic material, deformation is easy even by a small stress in the blood vessel since the particle is a porous one, so that it can cope with various blood vessel diameters. However, since porosity control is difficult, there is a problem that it is difficult to homogenize the amount of impregnation per each particle unit.

Moreover, Patent Document 2 discloses porous or non-porous granular product of a crosslinked gelatin and there is a description that it is useful in an embolization use. By thermal crosslinking of the gelatin granular product, it is intended to control dissolution time in physiological saline to 240 hours or less to thereby regulate embolization time in a blood vessel. However, swelling ability and the like of the particle one in physiological saline is not investigated at all.

Furthermore, Patent Document 3 discloses a blood vessel embolization agent using a spherical crosslinked gelatin particle, and dry particle diameter and swollen volume in physiological saline are described. However, since the spherical crosslinked gelatin disclosed herein is a particle chemically crosslinked using an aliphatic dial represented by glutaraldehyde as a crosslinking agent, the particle swells to such an extremely large degree as a swollen volume of 5 to 100 ml/g. In the case of the particle that swells to such a large degree, the mechanical strength of the swollen particle exceedingly decreases and there is a possibility that problems occur in view of firmness (mechanical strength and shape retention ability). Also, in the case where a gelatin particle subjected to a crosslinking treatment using a crosslinking agent is administrated to a living body, there arises a necessity of considering toxicity and the like of the crosslinking agent remaining in the gelatin particle, and hence there is a limitation on usable crosslinking agents.

Background Art Documents

Patent Document 1 : Japanese Patent No. 3879018
Patent Document 2 : JP-A-2010-83788
Patent Document 3 : JP-A-60-222045

SUMMARY OF THE INVENTION

In the case of a particle composed of gelatin or collagen, when the particle has a porous form, since a large amount of an impregnating solution such as physiological saline is absorbed into a porous part (void part) even when the impregnating solution is absorbed, the particle diameter hardly changes and the retained amount of the impregnating solution is large. However, in the case of a porous particle, the particle shape (particle diameter and the like) is maintained even when an impregnating solution is absorbed but the particle is easily deformed or crushed by the application of an external stress.

Moreover, in the case of administration of a gelatin particle to a living body, usually, the gelatin particle is charged into a dispersing solution such as physiological saline and is used in a suspended state. In this case, a shape advantageous in suspending ability and administration ability into the body is a particle shape, and it is judged that it is preferable to use not an indeterminate particle but a spherical to almost spherical particle sentient of circularity, from the viewpoints of homogeneous absorbability of a physiologically active substance and the like, ability of controlled release of the physiologically active substance upon administration, and homogeneous degradability of the particle in the body.

Furthermore, since it becomes possible to regulate a degradation rate of the gelatin particle in the living body by subjecting the gelatin particle to an insolubilization treatment, embolization time can be regulated in the case of the use for blood vessel embolization and the release of a physiologically active substance in the living body can be controlled in the case of the use for drug delivery to an affected part after the gelatin particle is impregnated with the physiologically active substance. However, in the case where the gelatin particle is administrated to the living body, a microcatheter or an injection needle is usually used but a gelatin particle larger than the diameter thereof cannot pass through it to clog it or, even when the particle passes through it, the particle is deformed during the passage and there is a concern that the particle becomes in a deformed state even after the passage or the gelatin particle is broken during the passage. In the case of the use in a blood vessel embolization use, when the gelatin particle is broken in the blood vessel, there is a concern that the gelatin particle is not kept at the target site for embolization but flows into a peripheral blood vessel and also there is a concern that the degradation time in the living body is shortened, so that it is considered that the time for controlled release cannot be controlled in the drug delivery use.

Based on the above, in order to obtain a gelatin particle that has not a porous form but a non-porous form and is excellent in retention ability of an impregnating solution and in firmness even upon swelling, investigation on swelling ability has been extensively studied. Further, with regard to the swollen particle, in order to obtain a swollen particle of gelatin that has an appropriate mechanical strength and also is excellent in deforming ability under stress, strength of gelatin that forms the particle has been also extensively studied.

As a result, the swelling of the particle proceeds concentrically on dry particle, and the particle swells to a certain particle diameter that is an equilibrium state. The swelling ratio at that time varies depending on the particle diameter of the dry particle, crosslinking conditions, and the components and solute concentration of the impregnating solution. In order to control the swelling ratio in usually used physiological saline, it has been found that it is important to control crosslinking temperature and crosslinking time that are conditions at thermal crosslinking of the gelatin particle and the degree of vacuum. Furthermore, when jelly strength of the gelatin used is controlled to a certain range, it has been found that a swollen gelatin particle obtained by swelling a thermally crosslinked gelatin particle to a specific particle diameter range is excellent in deformation-restoring ability against stress deformation.

For solving the problems in the above-mentioned background art, the present inventors have first focused their attention on gelatin excellent in biocompatibility and biodegradability as a main component that forms the particle and have performed investigation in order to obtain a gelatin particle most suitable for blood vessel embolization and for controlled release of a physiologically active substance. As a result, they have found that a gelatin particle that solves the above various problems can be obtained by controlling an average volume swelling ratio of a thermally crosslinked gelatin particle that has not a porous form but a non-porous form and is close to almost perfect sphere, and thus they have accomplished the invention.

Namely, the present invention provides the followings.

1. A gelatin particle, which is a thermally-crosslinked non-porous spherical gelatin particle having a circularity of 0.8 or more and a dry particle diameter of 20 to 1,600 $\mu$m, wherein an average volume swelling ratio in a case where the gelatin particle is immersed in physiological saline at 23°C is from 200 to 2,000% relative to an average volume of the gelatin particle in a dried state.

2. The gelatin particle according to item 1, wherein the gelatin particle is thermally crosslinked by subjecting the gelatin particle in a dried state to a heating treatment under vacuum.

3. A gelatin particle obtained by equilibrium swelling of the gelatin particle according to item 1 or 2 with an impregnating solution.

4. The gelatin particle according to item 3, wherein the impregnating solution is physiological saline.

5. The gelatin particle according to item 3, which is a swollen gelatin particle obtained by swelling the thermally-crosslinked non-porous spherical gelatin particle comprising gelatin having a jelly strength of 80 to 120 g with the impregnating solution, wherein the swollen gelatin particle has a particle diameter of 50 to 2,000 $\mu$m.

6. The gelatin particle according to item 5, wherein the swollen gelatin particle is restored from deformation to an almost spherical shape in a case where the swollen gelatin particle is deformed by applying a compression stress of a test strength of 20 mN at a loading rate of 1.1155 mN/second with a load retention time of 0 second and subsequently the compression stress is released.

7. The gelatin particle according to item 1, which is a gelatin particle for blood vessel embolization.

8. A gelatin particle for controlled release of physiologically active substance, which comprises the gelatin particle according to item 1 and a physiologically active substance dissolved and retained in the gelatin particle.

9. A device for administration of physiologically active substance,

wherein the gelatin particle for controlled release of physiologically active substance according to item 8 is dispersed and filled together with physiological saline into a syringe and used.

Particularly, the gelatin particle of the invention is preferably used in a blood vessel embolization use and a use for controlled release of a physiologically active substance with dissolving and retaining the physiologically active substance and as a device for administration into a living body wherein the gelatin particle for controlled release of a physiologically active substance is dispersed and filled together with physiological saline into a syringe and used.

Since the gelatin particle of the invention has not an indeterminate form such as a porous form but a non-porous spherical form having a circularity of 0.8 or more, the particle is excellent in firmness (shape retentionability) even when it swells with absorbing an impregnating solution such as physiological saline. Namely, because of excellent firmness, even when an external stress is applied to the particle after swelling, not only the ability of retaining the impregnating solution is excellent but also the particle is not broken. Thus, in the case of administration into a living body, particularly into a blood vessel, there can be prevented a risk that the broken fragmented gelatin particle flows out to a site other than the target site.

Moreover, the swollen gelatin particle in the invention does not have an indeterminate form such as a porous form and the particle is excellent in firmness even when it swells through absorption of an impregnating solution such as physiological saline. Namely, since gelatin having a specific jelly strength is used, the particle is excellent in firmness and, even when an external stress is applied to the particle after swelling and the particle is deformed, the particle is not broken. Thus, even in the case of administration into a living body, particularly into a blood vessel using a microcatheter or an injection needle as a particle for embolization, the particle can smoothly pass through them and also is excellent in stress deformation-restoring ability, so that there can be prevented a risk that the broken fragmented gelatin particle flows out to a site other than the target site.

Furthermore, the gelatin particle and the swollen gelatin particle of the invention are excellent in biocompatibility and also, since the particles are thermally crosslinked using no crosslinking agent, they not only have a high safety but also are excellent in biodegradability, so that they can be not only used in a temporary blood vessel embolization use but also impregnated with various physiologically active substances and administrated into a living body as a support for a drug delivery system (DDS) or a scaffolding material in the living body. Additionally, since the particle diameter at equilibrium swelling can be made constant by setting the average volume swelling ratio at the time of immersion in physiological saline to a specific range, the adsorbed amount and ability for controlled release of the physiologically active substance can be regulated per gelatin particle unit.

BRIEF DESCRIPTION OF THE DRAWINGS

[Fig. 1] Fig. 1 shows a microscopic image of the gelatin particle of Example 1 in a dried state.

[Fig. 2] Fig. 2 shows a microscopic image after the gelatin particle of Example 1 is immersed in physiological saline and swelled.

[Fig. 3] Fig. 3 shows a microscopic image of the gelatin particle of Comparative Example 1 in a dried state.

[Fig. 4] Fig. 4 shows a microscopic image after the gelatin particle of Comparative Example 1 is immersed in physiological saline and swelled.

[Fig. 5] Fig. 5 is a graph showing relationships between the particle diameter and heating temperature of dry particle and the average volume swelling ratio with regard to Examples 1 to 16.

[Fig. 6] Fig. 6 shows microscopic images of the state of the gelatin particle after passage through a catheter with regard to the products of Example 1 and Comparative Example 1.

[Fig. 7] Fig. 7 is a microscopic image showing the state of embolization of blood vessel of canine liver with the swollen particle of the gelatin particle of Example 1.

DETAILED DESCRIPTION OF THE INVENTION

With regard to the gelatin to be used in the invention, the kind (source) thereof is not particularly limited. For example, gelatin derived from bovine bone, bovine skin, swine bone, swine skin, and the like can be used but, from the viewpoint of safety in a living body, gelatin derived from swine bone or swine skin is preferably used.

Moreover, with regard to the jelly strength of gelatin, it is desirable to use gelatin having a jelly strength of 80 to 120 g, preferably 85 to 110 g. Incidentally, the "jelly strength" in the present invention is one measured in accordance with JIS K 6503. When the jelly strength is too low, the mechanical strength of gelatin insolubilized by thermal crosslinking and swelled is insufficient, the deformation-restoring ability after deformation by an external stress becomes poor, and a possibility of being broken by the external stress becomes high. On the other hand, when the jelly strength is too high, the thermally crosslinked gelatin particle has a decreased swelling ability and also the deformation by a stress is insufficient, namely, a possibility of clogging without sufficiently following the inner wall of a microcatheter becomes high at the time when the particle passes through the catheter having a diameter smaller than the diameter of the swollen particle.

The gelatin particle of the invention has not a porous form but a non-porous form (solid form) and is a spherical or almost spherical particle. When the particle has a porous form, a sufficient mechanical strength cannot be maintained in the blood vessel embolization use and there is a possibility that the particle undergoes shape deformation when an external stress is applied thereto and hence a blood vessel cannot be efficiently embolized. Moreover, in the case where the gelatin particle is used as a base material for controlled-release preparations, a physiologically active substance falls out and the retention of the physiologically active substance becomes insufficient, so that delivery of a predetermined amount of the physiologically active substance to a target site tends to be difficult. Therefore, in the case where the gelatin particle of the invention is used in uses such as blood vessel embolization and base materials for controlled release, it is necessary to use non-porous one having no pore.

For example, in the case where the gelatin particle of the invention is used in the embolization therapy use, the embolization of the target place is surely achieved by using a non-porous gelatin particle having a particle diameter corresponding to the inner diameter of the embolization site of a blood vessel and also damage of the inner wall of the blood vessel can be prevented owing to the spherical shape, so that a patient can be also relieved of pain. Furthermore, in the case of the non-porous gelatin particle, it gradually dissolves from the outer periphery of the swollen gelatin particle but, in the case of a porous gelatin particle, upon dissolution and degradation in the embolized blood vessel, a part of the gelatin particle separates and falls out to form fine particles in some cases and there is a concern that the fine particles are carried through a blood flow to embolize a blood vessel other than that at the target site. On the other hand, in the case of the non-porous gelatin particle, there is an advantage that the possibility of occurrence of such a problem is little.

Moreover, when the shape is an indeterminate shape, there is a concern of occurrence of problems that a homogeneous impregnation cannot be secured in the case of impregnation with a physiologically active substance or unevenness in a time-based released amount of the physiologically active substance used for impregnation occurs at the time when the gelatin particle gradually dissolves. Since the surface area per particle becomes almost constant in the case where the particle is a spherical one as in the invention, the homogeneous impregnation with the physiologically active substance and the homogeneous controlled release at the target site can be secured, so that the case is preferable. The spherical shape or almost spherical shape in the invention means that circularity of a circle formed when a particle is projected is 0.8 or more, and particles having a circularity of 0.8 or more account for preferably 70% by weight or more, particularly preferably 80% by weight or more of the whole particles. In the case of a particle having a circularity of less than 0.8, it is highly probable that particles become indeterminate ones including a lot of aggregated particles and attached particles, so that the particle is not suitable for use. Incidentally, the circularity in the invention is a value obtained by binarization image processing after a particle is projected as above, is obtained according to the formula of $4\pi S/L^2$ from the area (S) and circumference (L) of the circle when the particle is projected, and means a value approaching to 1 as the projected circle gets close to perfect circle.

The above gelatin particle desirably has a particle diameter in a dried state of 20 to 1,600 $\mu$m, preferably 25 to 600 $\mu$m, particularly preferably 40 to 250 $\mu$m from the viewpoint of therapeutic procedure at the administration into a living body. In the case of the particle diameter of less than 20 $\mu$m, there is a concern that the particle reaches a peripheral blood flow that is not a target site in some cases to form a blood clot when administrated into a living body, so that the case is not preferable. Moreover, in the case of the particle having a particle diameter exceeding 1,600 $\mu$m, such a consideration that the diameter of an injection needle or a microcatheter to be used at the administration into a living body should be adjusted to the particle diameter becomes necessary and also there is a possibility that invasiveness increases and hence the administration becomes burden to a patient, so that the case is not preferable. The gelatin particle of the invention can be used in various uses as long as it has a particle diameter within the above range. However, for example, in the case of uses in which the particle is allowed to pass through a syringe, a catheter, or the like, when the inner diameter of the catheter or syringe, the administration site, and the like are considered, in order to narrow the particle distribution of the gelatin particle to be used, it is preferable to sieve the prepared gelatin particle into various sizes before use. For example, in the case of a particle for blood vessel embolization, it is practical to classify the particle into sizes having a range of 25 to 63 $\mu$m, a range of 75 to 150 $\mu$m, a range of 212 to 300 $\mu$m, a range of 425 to 600 $\mu$m, and the like.

Moreover, the gelatin particle after swelling desirably has a particle diameter in a swelled state of 50 to 2,000 $\mu$m, preferably 70 to 1,000 $\mu$m from the viewpoint of therapeutic procedure at the administration into a living body. In the case of the particle diameter of less than 50 $\mu$m, for example, when used in the embolization therapy use, there is a possibility that the particle migrates to a site other than a target site and, for example, it clogs a peripheral blood vessel to cause hematogenous disorder, so that the case is not preferable. Moreover, in the case of the particle having a particle diameter exceeding 2,000 μm, a so-called skinning phenomenon occurs on the particle surface when the gelatin particle is prepared and hence it becomes difficult to remove oil and fat, various organic solvents, and the like remaining in the particle completely even using a method such as washing, drying, and the like and also a desired spherical gelatin particle tends to be difficult to obtain.

Incidentally, since a gelatin particle having a large dry particle diameter is suitable for uses in which a large amount thereof is used in a wide range, such as bone regeneration therapy, it is not suitable for uses in which the particle is used through a narrow tube such as an injection needle. Also, as a shape, it is not necessary to use spherical one such as the particle of the invention, and gel-like gelatin and sheet-like gelatin are suitable.

In order to obtain swollen gelatin particle as above, the particle diameter of the dry particle before swelling is preferably from about 20 to 1,600 μm although it depends on the degree of thermal crosslinking. The swollen gelatin particle of the invention can be used in various uses as long as it has a swollen particle diameter within the above range. However, for example, in the case of uses in which the particle is allowed to pass through a syringe, a catheter, or the like, when the inner diameter of the catheter or syringe, the administration site, and the like are considered, in order to narrow the particle distribution of the gelatin particle to be used, it is preferable to sieve the produced gelatin particle into various sizes. Specifically, in the case of a particle for blood vessel embolization, it is practical to classify the particle after swelling into sizes so that the particle diameter thereof falls within a range of 50 to 130 μm, a range of 150 to 300 μm, a range of 425 to 2,000 μm, or the like.

The swollen gelatin particle in the invention has a spherical shape but has a property of restoring deformation to an almost spherical shape at the time when the particle is relieved of the stress after the particle is deformed by applying a compression stress. Specifically, the particle is restored to an almost spherical shape at the time when it is deformed by applying a compression stress of a test strength of 20 mN at a loading rate of 1.1155 mN/second with a load retention time of 0 second and is then relieved of the stress.

Furthermore, the gelatin particle of the invention is subjected to a crosslinking treatment for insolubilization toward aqueous solvents, and not commonly used crosslinking with a crosslinking agent but thermal crosslinking by a heating treatment is performed. Namely, in the case of the crosslinking treatment with a crosslinking agent, since a reaction product (residue) of the crosslinking agent is bonded to the gelatin particle, toxicity and the like should be carefully investigated in the case where the gelatin particle is used in a living body, so that usable crosslinking agents are also limited. However, in the case where thermal crosslinking is performed by the heating treatment, there is absent any concern like the above, the crosslinking treatment becomes milder than the crosslinking treatment with the crosslinking agent, and the control of the degree of crosslinking is facilitated. Therefore, in the case of the gelatin particle subjected to the thermal crosslinking as in the invention, adjustment of the degree of swelling at the time when the particle is immersed in an aqueous solvent such as physiological saline and the control of a time required for complete dissolution also become easy.

In the invention, for the thermal crosslinking treatment, gelatin particle obtained by a known granulation method such as a W/O dispersion method, a microreactor method, a spray dry method, a spray freeze-dry method, or a pulverization method is once dried by means of a drying method such as blow drying, air drying, vacuum drying, or freeze drying and subsequently heated and dried at a temperature range of 100 to 200°C, preferably 120 to 180°C, for 2 to 48 hours, preferably 2 to 8 hours, whereby the gelatin particle can be thermally crosslinked. However, in the case of heating and drying under standing conditions, it is preferable to perform the heating treatment at a temperature range of 130 to 170°C±5°C for about 4 to 5 hours. On this occasion, drying may be performed under normal pressure but, since gelatin that is intrinsically water-soluble is easily influenced by oxygen, moisture, and the like in the air, a heating treatment under vacuum is preferable for performing homogeneous thermal crosslinking with good reproducibility. The "vacuum" in the invention means a pressure condition (degree of vacuum) of 10 kPa or less which can be achieved by a usual vacuum drier, using absolute vacuum (0 kPa) as a basis.

The following will specifically describe a process for producing the gelatin particle of the invention using a W/O dispersion method as one example of the granulation method. An outline of the gelatin particle production includes a liquid droplet-forming step of dispersing an aqueous gelatin solution into an oil and fat to form liquid droplets of the aqueous gelatin solution, a gelling step of cooling the liquid droplets of the gelatin liquid to effect gelling, a dehydration step, a washing step, a drying step, and a crosslinking step.

First, liquid droplets of the aqueous gelatin solution are formed in the liquid droplet-forming step. Specifically, gelatin is charged into water at about 0°C and the gelatin is homogeneously dissolved in water by means of a stirrer, a shaker, or the like to prepare an aqueous gelatin solution. Gelatin can be dissolved for a short period of time by warming the water to about 40 to 60°C on this occasion, so that the warming is preferable. The gelatin concentration of the aqueous gelatin solution thus obtained is set to a range of 2 to 20% by weight, preferably 5 to 15% by weight for easily obtaining gelatin particles having a narrow particle size distribution with various particle diameters by adjusting the viscosity of the liquid droplets to be formed.

In the liquid droplet-forming step, the aqueous gelatin solution prepared as mentioned above is dispersed into an oil and fat to form liquid droplets. For example, an excess amount of the oil and fat is charged into a flask fitted with a stirring blade, the aqueous gelatin solution prepared as mentioned above is charged under stirring, and the whole is stirred for an arbitrary time to disperse the aqueous gelatin solution homogeneously in the oil and fat in a liquid droplet state. Gelatin can be homogeneously dispersed with preventing solidification of the oil and fat by warming the oil and fat to 0 to 60°C on this occasion and also denaturation of gelatin can be prevented, so that the warming is preferable.

As the oil and fat for charging the aqueous gelatin solution therein, it is necessary to use an oil and fat having a poor compatibility with the aqueous gelatin solution for preparing the liquid droplets of the aqueous gelatin solution and, for example, at least one kind selected from the group consisting of animal oils, plant oils, mineral oils, silicone oils, fatty acids, fatty acid esters, and organic solvents can be used. Of these, preferably, it is desirable to use caprylic triglyceride, olive oil, isocetyl isostearate, cetyl 2-ethylhexanoate, and the like from the viewpoint of non-toxicity against the human body.

The gelling step is performed subsequently to the above liquid droplet-forming step. In the gelling step, the gelatin liquid droplets are gelled by cooling the liquid droplets of the aqueous gelatin solution. Here, the gelling means such a non-flowable state that the spherical shape of the liquid droplet can be maintained in the case where the gelled gelatin liquid droplets are taken out of the oil and fat through filtration. For gelling the gelatin in the liquid droplets by a cooling operation in the gelling step, it is sufficient to cool the oil and fat containing the liquid droplets dispersed therein. For example, in the case where the gelatin liquid droplets dispersed in the oil and fat are formed in a flask, it is sufficient to immerse the flask in a cooling water to cool the internal oil and fat and the gelatin liquid droplets through the outer wall of the flask. With regard to the setting of cooling temperature, for sure gelling, it is preferable to set the temperature to a range of 0 to 3°C. Namely, by cooling the gelatin liquid droplets within the temperature range, the water in the liquid droplets is not solidified and also the mechanical strength of the gelled gelatin liquid droplets is improved. As a result, deformation of the gelatin particle in the dehydration step and washing step to be mentioned later decreases and the oil and fat on the surface is easily washed in the washing step that is a post-step.

The cooling time in the above gelling step is from 15 to 90 minutes, preferably from about 30 to 60 minutes after the temperature reaches the set temperature of 0 to 3°C. When the cooling time is too short, the gelling of the gelatin liquid droplets tends to be insufficient. On the other hand, when the time is too long, the gelling has already sufficiently proceeded and hence time is wasted and production efficiency tends to be worse.

There is a concern that the oil and fat to be used in the above gelling step is solidified or viscosity of the oil and fat increases depending on the kind thereof by the temperature decrease through the cooling operation. In that case, these problems can be prevented by adding a dehydrating solvent having a low solidifying point and a low solution viscosity, so that the addition is preferable.

After gelling the gelatin liquid droplets by the cooling operation as mentioned above, water in the liquid droplets is removed in the dehydration step. Specifically, the dehydration step is performed by charging a dehydrating solvent under cooling at gelling temperature of the gelatin or lower (specifically, 15°C or lower) so that the gelled gelatin particle is not dissolved, and the water in the gelled gelatin particle is replaced therewith. Perfect removal of the water is achieved in the drying step to be mentioned later but the dehydration step aims at the replacement of the water in the gelled liquid droplets with the dehydrating solvent. Usually, the dehydration step is preferably performed for 15 minutes or more. By removing the water in the gelatin particle by replacing the water with the dehydrating solvent in the dehydration step, a homogeneous intra-particle crosslinking can be performed in the crosslinking step to be mentioned later and aggregation of the resulting gelatin particles themselves can be prevented.

In the dehydration step, since it is necessary to achieve dehydration by replacing the water in the liquid droplets with the dehydrating solvent as mentioned above, as the dehydrating solvent to be used, it is desirable to use a hydrophilic solvent having a solubility toward water of at least 10% by weight, preferably 30% by weight or more. As specific dehydrating solvents, for example, acetone, isopropyl alcohol, ethyl alcohol, methyl alcohol, tetrahydrofuran, and the like can be used singly or two or more thereof can be used in combination. Of these dehydrating solvents, from the viewpoint of easiness of replacement with water, it is suitable to use a hydrophilic organic solvent that is relatively freely mixable with water, such as acetone, isopropyl alcohol, or tetrahydrofuran.

Next, the washing step is performed subsequently to the above-described dehydration step. The cooled and gelled gelatin particle contains the dehydrating solvent that has been replaced with water in the dehydration step but the oil and fat and the like attached on the gelatin particle surface can be removed through the washing step. As the washing solvent to be used in the washing step, specifically, it is preferable to use an organic solvent in which the gelled gelatin particle is not dissolved. As in the case of the dehydrating solvent, the washing solvent is charged under cooling to the gelling temperature of the gelatin or lower. At the washing operation, washing is preferably repeated multiple times using a method such as filtration or centrifugation in combination. For example, in the case where about 2 to 15 g of the gelatin particle is washed, perfect washing is achieved by repeating an operation of washing for 15 to 30 minutes using about 200 to 300 ml of the washing solvent about four to six cycles.

As specific washing solvents, solvents the same as the above-described dehydrating solvents can be used but, from the viewpoint of sure dehydration of the gelatin particle, it is preferable to use ketone-based solvents such as acetone, alcohol-based solvents such as isopropyl alcohol, and tetrahydrofuran.

With regard to the gelatin particle obtained through the dehydration step and the washing step as mentioned above, for removing excess dehydrating solvent and washing solvent attached on the particle surface and completely removing water, the drying step is performed. As drying methods to be used in the drying step, for example, common drying methods such as blow drying, drying under reduced pressure (including vacuum drying), and freeze drying can be used. In the case of performing the drying under reduced pressure, it is preferable to perform drying at a temperature of about 5 to 25°C for about 6 to 12 hours, in order not to induce crosslinking of gelatin by heating.

The gelatin particle dehydrated and dried in the drying step is crosslinked in the crosslinking step. For example, in the case where the gelatin particle is introduced into the body as in the case of the embolization therapy use, since it is necessary to use a particle harmless to the human body, the adoption of the crosslinking method using an external crosslinking agent such as a crosslinking agent is not preferable and it is necessary to perform crosslinking by heating. In the case of the crosslinking by heating, for example, the gelatin particle can be crosslinked by heating at 80 to 250°C, preferably 130 to 170°C for 0.5 to 120 hours, preferably 3 to 24 hours. Incidentally, since the degree of crosslinking of the gelatin particle can be controlled by controlling the heating temperature and the heating time, a time required for complete dissolution in a aqueous solution or in a blood vessel (biodegradation time etc.) can be controlled.

In the case where it is intended to embolize a blood vessel using the dry gelatin particle obtained as above in the embolization therapy use, after the blood flow is blocked by blood vessel embolization using swollen gelatin particle obtained by immersing the gelatin particle in physiological saline or the like, a time until the blood flow is restored through gradual biodegradation of the swollen gelatin particle is controlled, that is, an embolization time can be controlled. Specifically, since blood vessel embolization for 2 to 3 days is sufficient for necrosis of a tumor such as liver cancer, it is preferable to control an embolization period until the blood flow is restored, i.e., a period until the swollen gelatin particle is biologically degraded to about 3 to 7 days in view of little damage to the organ and normal cells at the embolized site. Usually, in the case where the embolization period is set to 3 to 7 days, it is preferable to control the heating conditions in the crosslinking step to 100 to 180°C and 1 to 24 hours. Incidentally, since there is a concern that the gelatin particle is oxidized and denatured upon the heating operation in the crosslinking step, usually, the crosslinking step is preferably performed under reduced pressure or under an inert gas atmosphere.

In the case where the swollen gelatin particle obtained by swelling the gelatin particle of the invention is used in the blood vessel embolization use as mentioned above, for example, a catheter is inserted from the femoral artery or the like to a site to be intended to undergo embolization therapy with administrating a contrast agent, subsequently, a gelatin particle having an arbitrary shape is dispersed in physiological saline, a dispersion of the swollen gelatin particle subjected to equilibrium swelling is injected into the catheter, and the swollen gelatin particle is delivered to the target embolization therapy site, whereby the embolization can be achieved.

Moreover, the gelatin particle of the invention can be also used in the controlled release use of a physiologically active substance with impregnation and retention of the physiologically active substance inside the particle. In this case, for example, the particle can be used as a device for administration of a physiologically active substance, wherein the gelatin particle for controlled release of a physiologically active substance is dispersed and filled together with physiological saline into a syringe and used.

Incidentally, the above device for administration of a physiologically active substance in the invention is a device in a state of use. For example, the device also includes embodiments such as a case where the gelatin particle alone is filled into a syringe beforehand and physiological saline is sucked into the syringe at time of use to disperse the gelatin particle and a case where the gelatin particle is filled into a vial, physiological saline is charged at time of use to disperse the gelatin particle, and the dispersion is sucked into a syringe, i.e., a device for administration of a physiologically active substance obtained by so-called preparation at time of use.

As the physiologically active substance to be retained in the gelatin particle of the invention, there may be mentioned, in addition to drugs for treating various diseases, various growth factors such as basic fibroblast growth factor (bFGF), hepatocyte growth factor (HGF), and platelet-derived growth factor (PDGF), cytokines such as interferon and interleukin, and the like. Of these, for example, the gelatin particle retaining bFGF or the like can be used for treating lower extremity ischemic diseases.

The gelatin particle of the invention obtained as above has an average volume swelling ratio of 200 to 2,000%, preferably 400 to 800% relative to average volume of dry particle (gelatin particle in a dried state), in the case where it is immersed in physiological saline at 23°C. Swelling in physiological saline expands concentrically in the dry particle and reaches an equilibrium state in a range of the average volume swelling ratio of 200 to 600%. When the average volume swelling ratio is too small, that is, when swelling in physiological saline is too small, not only internal retention of an impregnating solution and a physiologically active substance becomes difficult but also a gel particle having a sufficient elasticity is not formed, so that the case tends to be an obstacle to combination use with medical devices in a procedure at therapy and hence is not preferable. On the other hand, when the average volume swelling ratio is too large, that is, when the particle drastically swells in physiological saline, mechanical strength as a swollen particle decreases and hence the decrease leads to particle collapse during the therapeutic procedure or particle collapse after administration into a living body, so that there is a concern that objective blood vessel embolization ability and performance as a base material for controlled release are not obtained and also there is a possibility that fine pieces resulting from the collapse mix into the blood flow to cause a blood clot at a place other than the target site.

Examples

The following will describe the invention more specifically with reference to Examples. However, the invention should not be construed as being limited to the description of Examples.

<Examples 1 to 16>

Using a medium-chain fatty acid glyceride as an oil and fat for dispersing gelatin, an aqueous gelatin solution (derived from swine skin, concentration: 5% by weight) was added dropwise and dispersed therein at 10°C or less to prepare a gelatin particle. Subsequently, the gelatin particle was cooled (0°C) under stirring to achieve thorough gelling and then acetone was added as a solvent to replace water in the gelatin particle therewith.

Then, after the gelatin particle was washed with acetone as a washing solvent, the particle was dried to obtain a non-porous spherical dry gelatin particle. The obtained gelatin particle was classified and recovered into four kinds of gelatin particles having particle diameters of 425 to 600 μm, 212 to 300 μm, 75 to 150 μm, and 25 to 63 μm. The four kinds of the classified and recovered gelatin particles are subjected to a heating treatment at a predetermined temperature for 4 to 5 hours in a standing state under a vacuum condition (5 kPa) to obtain gelatin particles of the invention (circularity: 0.9).

Heating temperature conditions for the prepared products of Examples are as follows.

Example 1: particle diameter of 425 to 600 μm, heating temperature: 150°C (±5°C)
Example 2: particle diameter of 212 to 300 μm, heating temperature: 150°C (±5°C)
Example 3: particle diameter of 75 to 150 μm, heating temperature: 150°C (±5°C)
Example 4: particle diameter of 25 to 63 μm, heating temperature: 150°C (±5°C)
Example 5: particle diameter of 425 to 600 μm, heating temperature: 130°C (±5°C)
Example 6: particle diameter of 212 to 300 μm, heating temperature: 130°C (±5°C)
Example 7: particle diameter of 75 to 150 μm, heating temperature: 130°C (±5°C)
Example 8: particle diameter of 25 to 63 μm, heating temperature: 130°C (±5°C)
Example 9: particle diameter of 425 to 600 μm, heating temperature: 140°C (±5°C)
Example 10: particle diameter of 212 to 300 μm, heating temperature: 140°C (±5°C)
Example 11: particle diameter of 75 to 150 μm, heating temperature: 140°C (±5°C)
Example 12: particle diameter of 25 to 63 μm, heating temperature: 140°C (±5°C)
Example 13: particle diameter of 425 to 600 μm, heating temperature: 170°C (±5°C)
Example 14: particle diameter of 212 to 300 μm, heating temperature: 170°C (±5°C)
Example 15: particle diameter of 75 to 150 μm, heating temperature: 170°C (±5°C)
Example 16: particle diameter of 25 to 63 μm, heating temperature: 170°C (±5°C)

<Comparative Example 1>

As a product of Comparative Example, a commercially available porous gelatin particle (trade name: Gelpart, manufactured by Nippon Kayaku Co., Ltd., particle diameter: about 1 mm) was used.

For the gelatin particles obtained in the above Examples and Comparative Example, the average volume swelling ratio in the case where each particle was immersed in physiological saline was measured as follows.
The particle diameter of each gelatin particle in a dry state and the particle diameter of each gelatin particle after immersed in physiological saline at 23°C for 30 minutes were observed on a microscope (model: VHX-500, manufactured by Keyence Corporation, lens: VH-Z100). The magnification at the observation on the microscope was 100× for particles having a particle diameter of 425 to 600 μm in a dry state and the product of Comparative Example (particle diameter: about 1 mm), 200× for particles having a particle diameter of 212 to 300 μm, 300× for particles having a particle diameter of 75 to 150 μm, and 700× for particles having a particle diameter of 25 to 63 μm. In the observation, 300 particles (n=300) were measured per sample and the average volume swelling ratio was calculated according to the following equation. The results are shown in Table 1.

$$[\text{Formula 1}] \quad \text{Average volume swelling ratio (\%)} = \{(\text{Average particle diameter after swelling}/2)^3/(\text{Average particle diameter at dry state}/2)^3\} \times 100$$

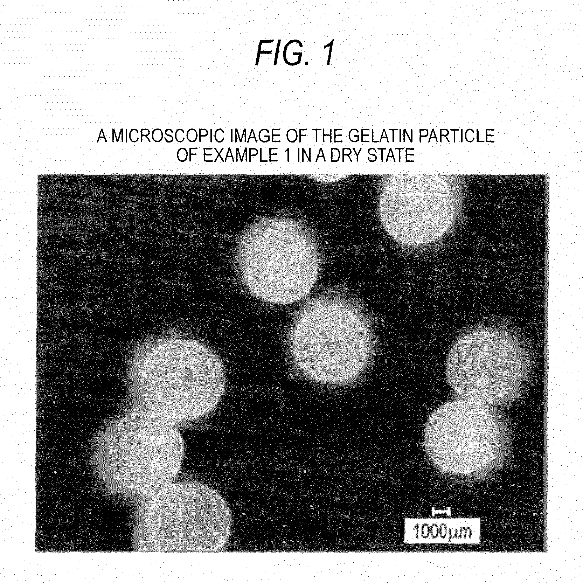

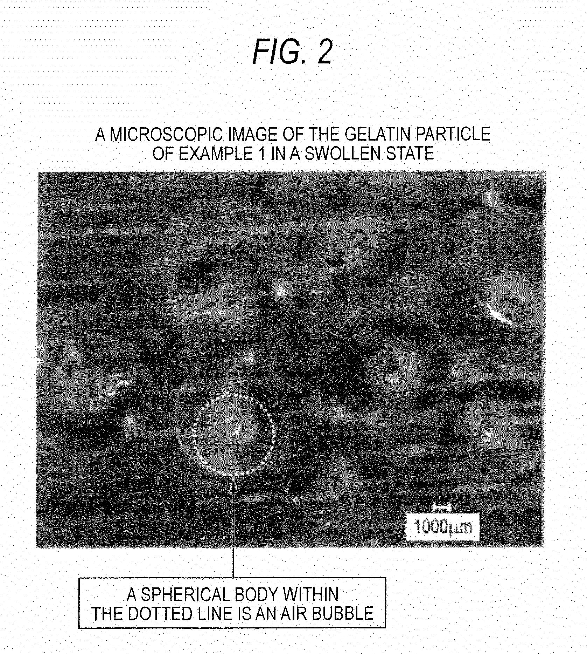

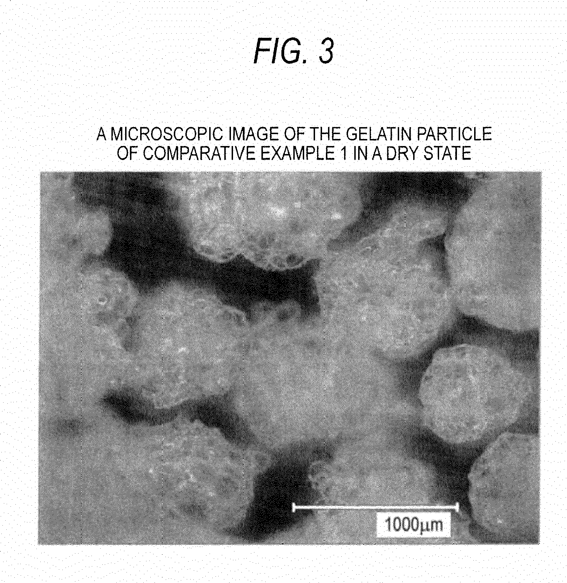

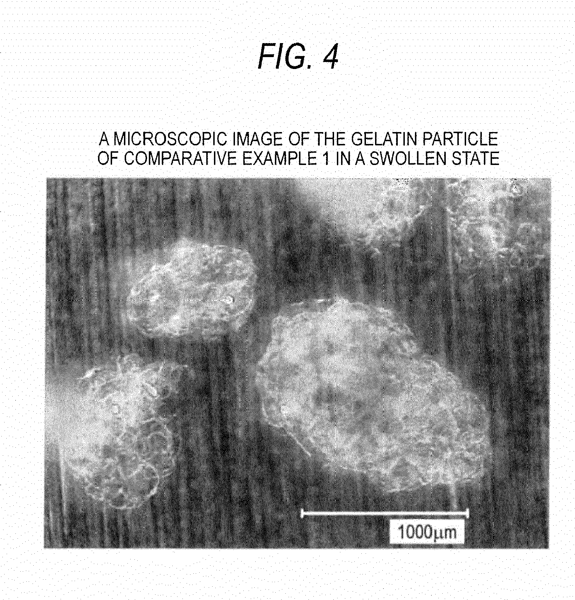

Fig. 1 to Fig. 4 show microscopic images of the gelatin particles of Example 1 and Comparative Example 1 at a dry state (Fig. 1, Fig. 3) and microscopic images after they were swelled by immersing them in physiological saline (Fig. 2, Fig. 4).

Table 1

| | Drying temperature | Particle state | Average particle diameter (μm) | Standard deviation (μm) | Maximum particle diameter (μm) | Minimum particle diameter (μm) | Average volume swelling ratio (%) |
|---|---|---|---|---|---|---|---|
| Example 1 | 150°C | Dry | 468 | 22 | 507 | 408 | 492 |
| | | Swollen | 796 | 39 | 870 | 683 | |
| Example 2 | | Dry | 231 | 10 | 261 | 202 | 580 |
| | | Swollen | 415 | 24 | 481 | 320 | |
| Example 3 | | Dry | 90 | 13 | 130 | 55 | 491 |
| | | Swollen | 153 | 19 | 205 | 103 | |
| Example 4 | | Dry | 44 | 10 | 74 | 29 | 476 |
| | | Swollen | 74 | 13 | 110 | 53 | |
| Example 5 | 130°C | Dry | 491 | 38 | 605 | 415 | 1305 |
| | | Swollen | 1156 | 86 | 1415 | 914 | |
| Example 6 | | Dry | 251 | 16 | 296 | 209 | 1319 |
| | | Swollen | 593 | 46 | 783 | 419 | |
| Example 7 | | Dry | 95 | 16 | 149 | 67 | 1401 |
| | | Swollen | 229 | 49 | 383 | 133 | |
| Example 8 | | Dry | 45 | 6 | 64 | 32 | 1944 |
| | | Swollen | 121 | 15 | 155 | 90 | |
| Example 9 | 140°C | Dry | 484 | 26 | 565 | 412 | 532 |
| | | Swollen | 845 | 47 | 988 | 705 | |
| Example 10 | | Dry | 246 | 12 | 290 | 214 | 689 |
| | | Swollen | 468 | 24 | 538 | 370 | |
| Example 11 | | Dry | 89 | 4 | 109 | 77 | 637 |
| | | Swollen | 165 | 9 | 221 | 135 | |
| Example 12 | | Dry | 38 | 6 | 59 | 28 | 1285 |
| | | Swollen | 89 | 15 | 142 | 63 | |

Table 1 (continued)

| | Drying temperature | Particle state | Average particle diameter ($\mu$m) | Standard deviation ($\mu$m) | Maximum particle diameter ($\mu$m) | Minimum particle diameter ($\mu$m) | Average volume swelling ratio (%) |
|---|---|---|---|---|---|---|---|
| Example 13 | 170°C | Dry | 486 | 22 | 540 | 387 | 257 |
| | | Swollen | 666 | 29 | 750 | 571 | |
| Example 14 | | Dry | 237 | 11 | 274 | 200 | 219 |
| | | Swollen | 308 | 16 | 385 | 253 | |
| Example 15 | | Dry | 87 | 5 | 102 | 66 | 256 |
| | | Swollen | 119 | 6 | 149 | 97 | |
| Example 16 | | Dry | 37 | 6 | 58 | 23 | 247 |
| | | Swollen | 50 | 8 | 77 | 35 | |
| Comparative Example 1 | - | Dry | 1106 | 202 | 1661 | 696 | 88 |
| | | Swollen | 1059 | 185 | 1587 | 694 | |

As is apparent from the results in Table 1, since the products of Examples of the present application had a non-porous spherical shape, the gelatin particles were swelled in the case where they were immersed in physiological saline but the product of Comparative Example having a porous indeterminate shape hardly swelled even when it was immersed in physiological saline.

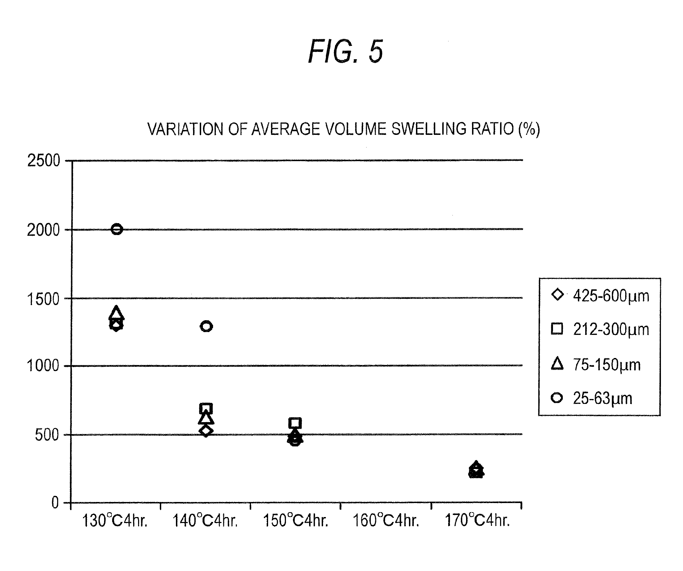

Furthermore, for Examples 1 to 16 in which the degree of crosslinking of the gelatin particles were altered by changing the heating temperature, relationships between the particle diameter and heating temperature of the dry particle and the average volume swelling ratio were summarized in Fig. 5. As is apparent from Fig. 5, it could be confirmed that, since the degree of crosslinking increased as the heating temperature was elevated regardless of particle diameter, the average volume swelling ratio tended to decrease. Incidentally, the products of Examples enabled control of the average volume swelling ratio in a range of 200 to 2,000%.

Moreover, in order to obtain a gelatin particle having an average volume swelling ratio of less than 200%, the degree of crosslinking was increased by controlling the heating temperature to 180°C or higher but yellowing of the gelatin particle became severe and thus the particle was not suitable for the embolization use and the use for controlled release of a physiologically active substance. On the other hand, in order to obtain a gelatin particle having an average volume swelling ratio of more than 2,000%, the degree of crosslinking was decreased by controlling the heating temperature to lower than 130°C but the swollen gelatin particle was swelled in physiological saline such a degree that the outline of the particle was not able to discriminate and also the mechanical strength of the particle decreased, so that a particle endurable for use could not be formed.

Next, catheter-passing ability of the gelatin particle of the invention after immersed in physiological saline was investigated to evaluate shape retention ability (physiological saline-retaining ability).
For the products of Example 1, Example 5, Example 9, Example 13, and Comparative Example 1 sieved into 425 to 600 μm after gelatin particle preparation, after each dry gelatin particle was immersed in physiological saline at 23°C for 30 minutes to achieve swelling, the swollen gelatin particle was sucked into a syringe, a pumping operation was performed 20 times with a three-way stopcock and a vacant syringe, and thereafter, the gelatin particle was poured through a catheter (product name: Prograde, manufactured by Terumo Corporation, 2.3 Fr. 110 cm, outlet diameter: 0.57 mmϕ).

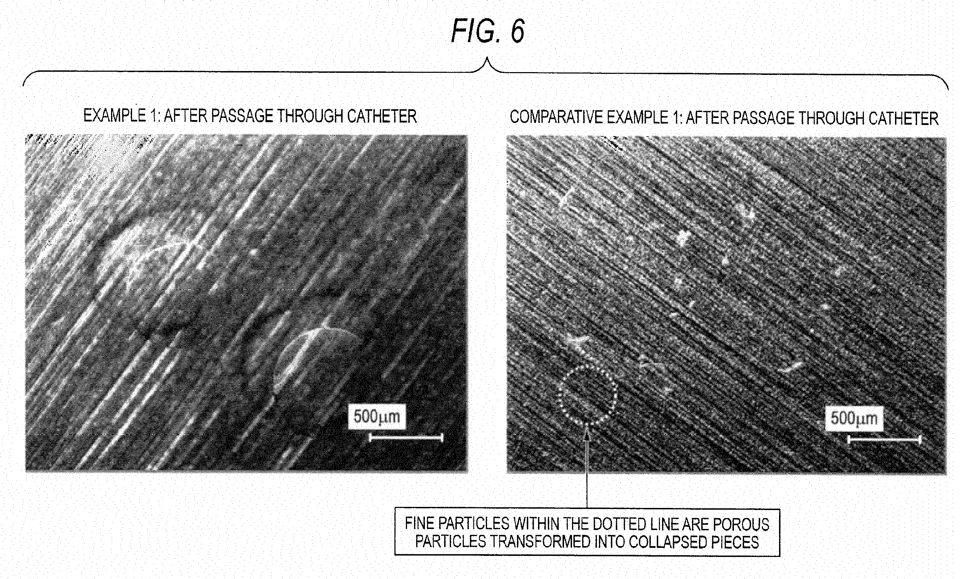

As above, the state that the swollen gelatin particle passed through the catheter was observed on a microscope. The physiological saline-retaining ability (shape retention ability) was judged as follows: the case where the volume change ratio of particle diameter after passage through catheter relative to the particle diameter before passage through catheter was 80% or more was judged as good, the case where the ratio was 50% or more and less than 80% was judged as moderate, and the case where the ratio was less than 50% was judged as bad. The results are described in Table 2. Also, Fig. 6 shows the state of each gelatin particle after passage through catheter for the products of Example 1 and Comparative Example 1.

As is apparent from Fig. 6, no change in particle diameter and particle shape was observed by the pumping operation performed before the passage through catheter in case of the product of Example 1 but the presence of a lot of collapsed pieces of the swollen gelatin particle were observed in the case of the product of Comparative Example 1.

Table 2

| | Passage through catheter | Average particle diameter ($\mu$m) | Standard deviation ($\mu$m) | Volume change ratio before and after passage through catheter (%) | Judgment (shape retention ability) |
|---|---|---|---|---|---|
| Example 1 | before passage | 796 | 39 | 103 | Good |
| | after passage | 804 | 54 | | |
| Example 5 | before passage | 1156 | 86 | 85 | Good |
| | after passage | 1097 | 94 | | |
| Example 9 | before passage | 845 | 47 | 108 | Good |
| | after passage | 866 | 27 | | |
| Example 13 | before passage | 666 | 29 | 107 | Good |
| | after passage | 681 | 21 | | |
| Comparative Example 1 | before passage | 1059 | 185 | 62 | Moderate |
| | after passage | 902 | 107 | | |

* The gelatin particles of Examples and Comparative example were regarded to have a spherical shape and the volume change ratio before and after passage through catheter was calculated according to the following equation.

Volume change ratio before and after passage through catheter (%) = {(Average particle diameter after passage/2)$^3$/(Average particle diameter before passage/2)$^3$} × 100

EP 2 708 570 A1

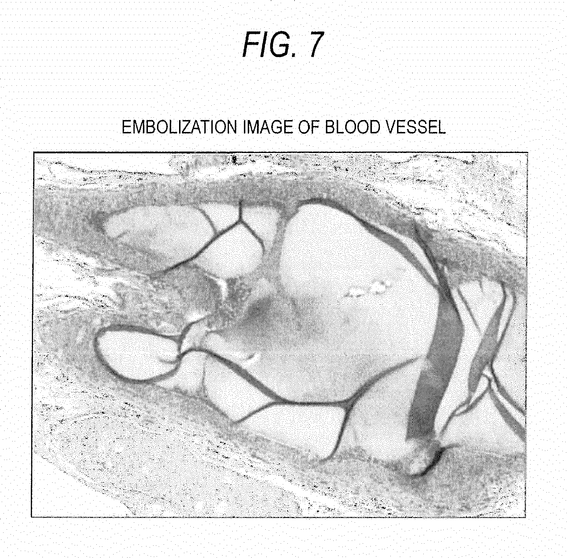

Next, using the swollen gelatin particle prepared in Example 1, an embolization test on canine liver was performed and it was observed on a microscope whether the particle followed the blood vessel wall at an embolization site to achieve embolization or not. Fig. 7 shows an image after 48 hours from the embolization of the blood vessel.

The central part in Fig. 7 is the gelatin particle. Thus, it could be confirmed that the gelatin particles of the invention had flexibility capable of following the blood vessel wall at the embolization site.

As is apparent from the above results, it was obvious that the gelatin particles of the invention showed swelling ability with physiological saline as an aqueous impregnating solution, firmness of the swollen particles is satisfactory even when the pumping treatment was performed, and the particles had flexibility.

<Example 17>

Into a 1,000 ml flask fitted with a stirring blade was charged 300 g of caprylic triglyceride as an oil and fat. Thereto was added 50 g of an aqueous gelatin solution (gelatin derived from swine skin having a jelly strength of 87g), and the whole was stirred at 120 rpm for 30 minutes to form liquid droplets of the aqueous gelatin solution in caprylic triglyceride.

Next, while caprylic triglyceride was cooled by immersing the flask in cold water at 5°C, the whole was stirred at 180 rpm for 60 minutes to cool the liquid droplets of the aqueous gelatin solution, thereby gelling the liquid droplets.

Then, 100 ml of ice-cooled (-10°C) acetone was added into the flask and the whole was stirred at 300 rpm for 30 minutes to achieve replacement and dehydration of water in the liquid droplets of the gelled gelatin with acetone, thereby obtaining a gelatin particle.

The obtained gelatin particle was separated by filtration, washed five times with an excess amount of ice-cooled acetone (-10°C) to remove caprylic triglyceride attached to the gelatin particle, and then dried under vacuum for 24 hours to obtain a gelatin particle from which the remaining solvent was removed.

Finally, after the obtained gelatin particle was classified, thermal crosslinking was performed at 150°C under vacuum (5 kPa) for 4 hours to obtain a thermally crosslinked non-porous spherical gelatin particle.

<Example 18>

A thermally crosslinked non-porous spherical gelatin particle was prepared in the same manner as in Example 17 except that the jelly strength of gelatin derived from swine skin in Example 17 was changed from 87 g to 104 g.

<Example 19>

A thermally crosslinked non-porous spherical gelatin particle was prepared in the same manner as in Example 17 except that the jelly strength of gelatin derived from swine skin in Example 17 was changed from 87 g to 117 g.

<Example 20>

A thermally crosslinked non-porous spherical gelatin particle was prepared in the same manner as in Example 17 except that the jelly strength of gelatin derived from swine skin in Example 17 was changed from 87 g to 104 g and the stirring rate at the formation of liquid droplets of the aqueous gelatin solution was changed from 120 rpm to 140 rpm.

<Example 21>

A thermally crosslinked non-porous spherical gelatin particle was prepared in the same manner as in Example 17 except that the jelly strength of gelatin derived from swine skin in Example 17 was changed from 87 g to 89 g and the stirring rate at the formation of liquid droplets of the aqueous gelatin solution was changed from 120 rpm to 160 rpm.

<Example 22>

A thermally crosslinked non-porous spherical gelatin particle was prepared in the same manner as in Example 17 except that the jelly strength of gelatin derived from swine skin in Example 17 was changed from 87 g to 104 g and the stirring rate at the formation of liquid droplets of the aqueous gelatin solution was changed from 120 rpm to 180 rpm.

<Example 23>

A thermally crosslinked non-porous spherical gelatin particle was prepared in the same manner as in Example 17 except that the stirring rate at the formation of liquid droplets of the aqueous gelatin solution was changed from 120 rpm to 550 rpm and the stirring rate at the cooling of liquid droplets of the aqueous gelatin solution was changed from 180 rpm to 550 rpm.

<Example 24>

A thermally crosslinked non-porous spherical gelatin particle was prepared in the same manner as in Example 23 except that the jelly strength of gelatin derived from swine skin in Example 23 was changed from 87 g to 104 g.

<Example 25>

A thermally crosslinked non-porous spherical gelatin particle was prepared in the same manner as in Example 23 except that the jelly strength of gelatin derived from swine skin in Example 23 was changed from 87 g to 117 g.

<Comparative Example 2>

A thermally crosslinked non-porous spherical gelatin particle was prepared in the same manner as in Example 17 except that the jelly strength of gelatin derived from swine skin in Example 17 was changed from 87 g to 68 g.

<Comparative Example 3>

A thermally crosslinked non-porous spherical gelatin particle was prepared in the same manner as in Example 17 except that the jelly strength of gelatin derived from swine skin in Example 17 was changed from 87 g to 135 g.

<Comparative Example 4>

A thermally crosslinked non-porous spherical gelatin particle was prepared in the same manner as in Example 21 except that the jelly strength of gelatin derived from swine skin in Example 21 was changed from 89 g to 60 g.

<Comparative Example 5>

A thermally crosslinked non-porous spherical gelatin particle was prepared in the same manner as in Example 21 except that the jelly strength of gelatin derived from swine skin in Example 21 was changed from 89 g to 150 g.

<Comparative Example 6>

A thermally crosslinked non-porous spherical gelatin particle was prepared in the same manner as in Example 23 except that the jelly strength of gelatin derived from swine skin in Example 23 was changed from 87 g to 68 g.

<Comparative Example 7>

A thermally crosslinked non-porous spherical gelatin particle was prepared in the same manner as in Example 23 except that the jelly strength of gelatin derived from swine skin in Example 23 was changed from 87 g to 135 g.

<Comparative Example 8>

A thermally crosslinked non-porous spherical gelatin particle was prepared in the same manner as in Example 21 except that the jelly strength of gelatin derived from swine skin in Example 21 was changed from 89 g to 217 g.

<Comparative Example 9>

A thermally crosslinked non-porous spherical gelatin particle was prepared in the same manner as in Example 21 except that the jelly strength of gelatin derived from swine skin in Example 21 was changed from 89 g to 292 g.
The gelatin particles of Examples and Comparative Examples prepared as above were immersed in physiological saline to achieve swelling and the passing ability through a microcatheter or an injection needle was confirmed by the method shown below and was described in Table 3. Furthermore, after the gelatin particles prepared in Example 21 and Comparative Examples 8 and 9 were swelled, 30% compression stress of each of the particles was measured by the following method and was collectively described in Table 3.

<Passing Ability of Swollen Particle through Microcatheter or Injection Needle>

The particle diameter of each of the dry particles prepared in Examples and Comparative Examples was measured for 50 pieces of the particle on a microscope (VHX-500, manufactured by Keyence Corporation, magnification: $100\times$ to $700\times$). The average particle diameter on that occasion was taken as a dry particle diameter.

After the dry particle was immersed in a stain solution (5% by weight aqueous solution of gentian violet) for 10 minutes to effect staining, the particle was immersed in physiological saline to perform an operation of washing an excess stain solution. The particle diameter of the particle swelled by immersion in physiological saline for 30 minutes in total was measured for 50 pieces of the particle on a microscope. The average particle diameter on that occasion was taken as a swollen particle diameter.

A microcatheter or injection needle having each inner diameter shown in Table 3 was connected to a syringe, and the gelatin particle swelled by the above operation was charged into the syringe together with the physiological saline and then was injected. The states of the swollen gelatin particle during the passage through the microcatheter or injection needle and after the passage were observed on the microscope and the passing ability of the swollen particle was judged.

Very good: the particle smoothly passed through the microcatheter or injection needle and the particle shape after passage was almost spherical one.

Good: the particle passed through the microcatheter or injection needle with slight clogging and the particle shape after passage was almost spherical one.

Moderate: the particle smoothly passed through the microcatheter or injection needle but the particle shape after passage was deformed or broken.

Bad: the particle clogged inside of the microcatheter or injection needle and hence was not able to pass through.

<30% Compression Stress>

The 30% compression stress of the gelatin particle swelled in purified water for 20 minutes was measured. For the measurement, a micro compression tester (MCT-211, manufactured by Shimadzu Corporation) was used and the measurement was performed under the following conditions.

Kind of pressurization indenter: FLAT500
Magnification of objective lens: $10\times$
Test mode: compression test
Test finishing condition: reaching set test strength
Test strength: 20 mN
Loading rate: 1 (1.1155 mN/second)
Load retention time: 0 second

Table 3

| | | Swollen particle diameter (µm) | Dry particle diameter (µm) | Jelly strength (g) | Passing ability through microcatheter or injection needle (mmφ) | | | | | 30% Compression stress (mN) | |
|---|---|---|---|---|---|---|---|---|---|---|---|
| | | | | | 1.70 | 0.57 | 0.33 | 0.13 | 0.08 | Average (n=3) | Standard deviation |
| Example | 17 | 1801 | 1299 | 87 | Good | - | - | - | - | - | - |
| | 18 | 1818 | 1242 | 104 | Good | - | - | - | - | - | - |
| | 19 | 1725 | 1242 | 117 | Good | - | - | - | - | - | - |
| | 20 | 850 | 479 | 104 | - | Very good | - | - | - | - | - |
| | 21 | 551 | 202 | 89 | - | - | Very good | | | 1.04 | 0.08 |
| | 22 | 162 | 85 | 104 | - | - | - | Very good | - | - | - |
| | 23 | 95 | 44 | 87 | - | - | - | - | Very good | - | - |
| | 24 | 83 | 45 | 104 | - | - | - | - | Very good | - | - |
| | 25 | 100 | 50 | 117 | - | - | - | - | Very good | - | - |
| Comparative Example | 2 | 1799 | 1230 | 68 | Moderate | - | - | - | - | - | - |
| | 3 | 1842 | 1189 | 135 | Bad | - | - | - | - | - | - |
| | 4 | 568 | 210 | 60 | - | - | Moderate | - | - | - | - |
| | 5 | 541 | 236 | 150 | - | - | Bad | - | - | - | - |
| | 6 | 94 | 44 | 68 | - | - | - | - | Moderate | - | - |
| | 7 | 89 | 49 | 135 | - | - | - | - | Bad | | |
| | 8 | 575 | 208 | 217 | - | - | Bad | - | - | 1.96 | 0.19 |
| | 9 | 555 | 202 | 292 | - | - | Bad | - | - | 3.03 | 0.36 |

From the result of Table 3, since the swollen gelatin particles of Examples all pass through the microcatheter or injection needle having a diameter smaller than the swollen particle diameter and return to an almost spherical shape after passage, it is obvious that the particles have restoring ability from stress and also are particles excellent in passing ability. On the other hand, the swollen gelatin particles of Comparative Examples 2, 4, and 6 were broken when they passed through the microcatheter or injection needle and they were still present in a broken or deformed state. It is considered that this is because the restoring ability from stress after deformation is low and thus shape restoration after deformation is not attained since the jelly strength of the gelatin forming the particle is less than 80 g. Moreover, in Comparative Examples 3, 5, and 7, the particles clogged inside of the microcatheter or injection needle when they passed through the microcatheter or injection needle. It is considered that this is because a stress at the time of deforming the swollen particles is too high since the jelly strength of the gelatin forming the particle exceeds 120 g. Furthermore, in Comparative Examples 8 and 9, the gelatin particles clogged inside of the microcatheter or injection needle and were impossible to pass through. It is surmised that this is because a high stress is imparted at the time when the swollen gelatin particles pass through the microcatheter or injection needle and a sufficient deformation cannot be achieved since the jelly strength of the gelatin is so large as 200 g or more.

Incidentally, even in the gelatin particles having a swollen particle diameter of 50 $\mu$m, when the jelly strength is from 80 g to 120 g, it is surmised that results similar to those of Examples are obtained. However, since the swollen diameter is smaller than the minimum diameter of microcatheters and injection needles currently commercially available for medical use, evaluation of passing ability was not carried out.

While the invention has been described in detail and with reference to specific embodiments thereof, it will be apparent to one skilled in the art that various changes and modifications can be made therein without departing from the scope thereof.

This application is based on Japanese patent application No. 2012-203806 filed on September 18, 2012 and Japanese patent application No. 2012-203807 filed on September 18, 2012, the entire contents thereof being hereby incorporated by reference.

## Claims

1. A gelatin particle, which is a thermally-crosslinked non-porous spherical gelatin particle having a circularity of 0.8 or more and a dry particle diameter of 20 to 1,600 $\mu$m, wherein an average volume swelling ratio in a case where the gelatin particle is immersed in physiological saline at 23°C is from 200 to 2,000% relative to an average volume of the gelatin particle in a dried state.

2. The gelatin particle according to claim 1, wherein the gelatin particle is thermally crosslinked by subjecting the gelatin particle in a dried state to a heating treatment under vacuum.

3. A gelatin particle obtained by equilibrium swelling of the gelatin particle according to claim 1 or 2 with an impregnating solution.

4. The gelatin particle according to claim 3, wherein the impregnating solution is physiological saline.

5. The gelatin particle according to claim 3, which is a swollen gelatin particle obtained by swelling the thermally-crosslinked non-porous spherical gelatin particle comprising gelatin having a jelly strength of 80 to 120 g with the impregnating solution, wherein the swollen gelatin particle has a particle diameter of 50 to 2,000 $\mu$m.

6. The gelatin particle according to claim 5, wherein the swollen gelatin particle is restored from deformation to an almost spherical shape in a case where the swollen gelatin particle is deformed by applying a compression stress of a test strength of 20 mN at a loading rate of 1.1155 mN/second with a load retention time of 0 second and subsequently the compression stress is released.

7. The gelatin particle according to claim 1, which is a gelatin particle for blood vessel embolization.

8. A gelatin particle for controlled release of physiologically active substance, which comprises the gelatin particle according to claim 1 and a physiologically active substance dissolved and retained in the gelatin particle.

9. A device for administration of physiologically active substance, wherein the gelatin particle for controlled release of physiologically active substance according to claim 8 is dispersed and filled together with physiological saline into a syringe and used.

# FIG. 1

A MICROSCOPIC IMAGE OF THE GELATIN PARTICLE
OF EXAMPLE 1 IN A DRY STATE

# FIG. 2

A MICROSCOPIC IMAGE OF THE GELATIN PARTICLE
OF EXAMPLE 1 IN A SWOLLEN STATE

1000μm

A SPHERICAL BODY WITHIN
THE DOTTED LINE IS AN AIR BUBBLE

# *FIG. 3*

A MICROSCOPIC IMAGE OF THE GELATIN PARTICLE
OF COMPARATIVE EXAMPLE 1 IN A DRY STATE

1000μm

# FIG. 4

A MICROSCOPIC IMAGE OF THE GELATIN PARTICLE
OF COMPARATIVE EXAMPLE 1 IN A SWOLLEN STATE

1000μm

# FIG. 5

VARIATION OF AVERAGE VOLUME SWELLING RATIO (%)

Legend:
- ◇ 425-600μm
- □ 212-300μm
- △ 75-150μm
- ○ 25-63μm

## FIG. 6

EXAMPLE 1: AFTER PASSAGE THROUGH CATHETER

COMPARATIVE EXAMPLE 1: AFTER PASSAGE THROUGH CATHETER

500μm

500μm

FINE PARTICLES WITHIN THE DOTTED LINE ARE POROUS
PARTICLES TRANSFORMED INTO COLLAPSED PIECES

EP 2 708 570 A1

# FIG. 7

EMBOLIZATION IMAGE OF BLOOD VESSEL

**Europäisches Patentamt**
**European Patent Office**
**Office européen des brevets**

# EUROPEAN SEARCH REPORT

Application Number

EP 13 18 4709

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,P | EP 2 596 858 A1 (NITTO DENKO CORP [JP]) 29 May 2013 (2013-05-29) * column 17 - column 21 * ----- | 1-9 | INV. C08H1/06 C08J3/24 A61K47/42 A61K9/16 |
| X | EP 2 168 557 A2 (NITTO DENKO CORP [JP]) 31 March 2010 (2010-03-31) * paragraph [0038] - paragraph [0047] * ----- | 1-9 | |
| X | JP 2010 083788 A (JELLICE CO LTD) 15 April 2010 (2010-04-15) * paragraph [0022] - paragraph [0025] * ----- | 1-9 | |
| | | | TECHNICAL FIELDS SEARCHED (IPC) C08H C08J A61K |

The present search report has been drawn up for all claims

| Place of search | Date of completion of the search | Examiner |
|---|---|---|
| Munich | 19 November 2013 | Friedrich, Christof |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 13 18 4709

This annex lists the patent family members relating to the patent documents cited in the above-mentioned European search report.
The members are as contained in the European Patent Office EDP file on
The European Patent Office is in no way liable for these particulars which are merely given for the purpose of information.

19-11-2013

| Patent document cited in search report | | Publication date | Patent family member(s) | | Publication date |
|---|---|---|---|---|---|
| EP 2596858 | A1 | 29-05-2013 | CN | 103025417 A | 03-04-2013 |
| | | | EP | 2596858 A1 | 29-05-2013 |
| | | | JP | 2012024313 A | 09-02-2012 |
| | | | US | 2013119570 A1 | 16-05-2013 |
| | | | WO | 2012011313 A1 | 26-01-2012 |
| EP 2168557 | A2 | 31-03-2010 | EP | 2168557 A2 | 31-03-2010 |
| | | | JP | 5307490 B2 | 02-10-2013 |
| | | | JP | 2010077062 A | 08-04-2010 |
| | | | US | 2010081790 A1 | 01-04-2010 |
| JP 2010083788 | A | 15-04-2010 | JP | 4422191 B1 | 24-02-2010 |
| | | | JP | 2010083788 A | 15-04-2010 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 3879018 B
- JP 2010083788 A
- JP 60222045 A
- JP 2012203806 A
- JP 2012203807 A